# FASCICULE DE BREVET EUROPEEN

(11) **EP 1 954 229 B1**
(45) Date de publication et mention de la délivrance du brevet: **18.09.2013**
(21) Numéro de dépôt: 06831347.7
(22) Date de dépôt: 16.11.2006
(51) Int. Cl.: A61F 13/02

(54) **PANSEMENT COMPRENANT UN APPLICATEUR DE FILM MINCE**
PFLASTER MIT APPLIKATOR ZUM AUFTRAGEN EINER DÜNNEN FOLIE
DRESSING PROVIDED WITH A THIN FILM APPLICATOR

(30) Priorité: 17.11.2005 FR 0511646
(43) Date de publication de la demande: 13.08.2008
(73) Titulaire: Laboratoires Urgo, 21300 Chenove (FR)
(72) Inventeur: PERNOT, Jean-Marc, F-21000 Dijon (FR)
(74) Mandataire: Milien, Jean-Baptiste Aurélien
(86) Numéro de dépôt international: PCT/FR2006/051185
(87) Numéro de publication internationale: WO 2007/057612

(56) Documents cités:
- EP-A- 0 051 935
- EP-A- 0 360 458
- WO-A-89/11262
- WO-A-95/25492
- WO-A-97/25012
- US-B1- 6 169 224

## Description

La présente invention concerne un nouveau système d'application de pansements.

### Art antérieur

Des pansements à films minces, généralement transparents, sont largement utilisés comme couche de protection par-dessus des plaies, car ils facilitent la cicatrisation dans un environnement humide, tout en agissant comme une barrière contre les liquides et la contamination par les bactéries. Ces films sont utilisés également comme champs chirurgicaux en raison de cette propriété de barrière contre la contamination bactérienne. Des pansements et des champs chirurgicaux tels que décrits ci-dessus sont commercialisés sous les dénominations suivantes : TEGADERM^{®} (société 3M, St. Paul, MN) décrit dans le brevet EP 51 935 et OPSITE^{®} (société T.J. Smith & Nephew, Hull, Angleterre).

Les films polymères utilisés dans ces pansements sont conformables, c'est-à-dire que les films sont suffisamment fins, flexibles et souples pour s'adapter de manière optimale à la topologie de la surface sur laquelle ils sont posés. Les films se présentent avant utilisation avec une couche de protection détachable recouvrant la surface enduite d'adhésif du film. Lorsque la couche est enlevée, le film enduit d'adhésif, encore appelé support, a tendance à faire des plis et à coller sur lui-même, empêchant ainsi une application aseptique et douce du pansement sur la peau d'un patient. Différents systèmes d'application de ces produits ont été proposés pour tenter de supprimer ce problème.

Le principe de ces différents systèmes consiste à ajouter sur le film enduit une couche supplémentaire d'un matériau rigide soit sous forme d'une couche uniforme tel un film soit sous forme d'un cadre, ce matériau rigide facilitant la pose et étant retiré après application du pansement sur la peau.

Toutefois ces solutions si elles favorisent, dans une certaine mesure, la pose entraînent l'apparition de nombreux problèmes lors du retrait de la couche de matériau rigide.

La différence de rigidité entre le film mince et cette couche supplémentaire qui est collée, thermocollée ou fixée par une liaison de nature mécanique sur la surface du film opposée à celle enduite d'adhésif entraîne lors du retrait de cette dernière une perturbation de la liaison adhésive entre la peau et le pansement. Ceci peut entraîner un décollement partiel du pansement de la peau ou l'apparition de plis qui peuvent provoquer au final un défaut prématuré d'adhérence du pansement soit une mauvaise application. Ainsi, dans le cas des pansements dans lequel il y a une couche rigide uniforme liée à la totalité de la face de dessus du support, il n'y a aucun moyen pour absorber la force exercée par le retrait de cette couche rigide dans un geste dans une seule direction ce qui altère la qualité de la liaison adhésive entre le pansement et la peau du patient.

De même, dans le cas des pansements dans lesquels la couche rigide est un cadre, lorsqu'il doit être détaché du périmètre du pansement, la force qui va s'appliquer lors du geste de décollement périphérique du cadre en contact avec le film mince entraîne elle aussi une altération de la qualité adhésive entre lé pansement et la peau.

Un autre inconvénient de ces pansements à cadre est qu'ils ne sont pas suffisamment rigides : après retrait de la couche de protection de l'adhésif, ceux-ci ont tendance à se recourber rendant la pose difficile.

Bien que ces deux grands types de pansements existent depuis de nombreuses années, aucune solution n'a été trouvée pour absorber la force imposée lors du retrait de la partie applicateur par la couche rigide.

### Objet de l'invention

C'est l'objet de la présente invention que de fournir un pansement qui permette d'absorber les forces exercées lors du retrait de l'applicateur sans altérer les propriétés de rigidité de l'ensemble pansement-applicateur lors de la pose sur la peau du patient.

Selon un premier aspect de l'invention, pour atteindre ce but, le pansement composite adhésif selon l'invention comprend :
- un support constitué d'un film mince et souple ayant une face de dessus et une face de dessous ;
- un adhésif sensible à la pression appliqué sur au moins une partie de la face du dessous du support ;
- une couche de protection appliquée de façon détachable sur l'adhésif sensible à la pression à l'opposé du support,
caractérisé en ce qu'il comporte en outre :
- une couche de liaison comprenant une face de dessus et une face de dessous qui est assemblée de façon détachable par sa face de dessous à la face de dessus du support à l'opposé de l'adhésif ; et
- des moyens de rigidification comprenant une face de dessus et une face de dessous, et étant fixés au moins en partie sur la périphérie du pansement sur la face de dessus de la couche de liaison par un moyen de fixation, en laissant la zone centrale de ladite couche de liaison non recouverte par lesdits moyens de rigidification, lesdits moyens de rigidification et ladite couche apportant une rigidité au support, la force de liaison entre le support couvert au moins en partie par l'adhésif et la peau étant supérieure à la force de liaison entre le support et la couche de liaison, et la force de liaison entre les moyens de rigidification et la couche de liaison étant elle aussi supérieure à la force de liaison entre la couche de liaison et le support.

On comprend que ce pansement composite de la présente invention résout ces problèmes en intercalant entre des moyens de rigidification, par exemple un cadre, et un film mince appelé support, une couche de liaison qui est en fait un second film comme, par exemple, un film mince et souple, qui sera éliminé en même temps que les moyens de rigidification et qui permet ainsi d'éviter les problèmes d'altération de la liaison adhésive entre la peau et le pansement.

Cette couche supplémentaire permet en effet de diminuer et d'absorber les forces qui s'exercent sur le pansement lors du retrait de la couche rigide et d'éliminer les problèmes précités dans les pansements utilisant comme moyen d'application une couche rigide et en particulier un cadre qui couvre la périphérie du pansement.

Le pansement selon la présente invention permet d'obtenir une force de décomplexage entre la couche de liaison et le film mince plus faible que celle des pansements existants (le pansement adhère à la peau sans risque de retrait lors du décomplexage du film mince et de la couche supplémentaire) tout en ayant un pansement plus rigide après retrait de la couche de protection de l'adhésif, facilitant ainsi la pose.

Ainsi, après retrait de la couche de protection couvrant l'adhésif, on fixe le pansement puis on élimine l'ensemble constitué des moyens de rigidification, par exemple un cadre, et de la couche de liaison pour obtenir une fixation sans problème du pansement final constitué du support et de l'adhésif. Ceci est réalisable car les moyens de rigidification et notamment le cadre permettent de rigidifier le pansement avant le dépôt et le film mince permet dans un geste simple, dans une seule direction, d'éviter les problèmes d'altération de la liaison entre la peau et l'adhésif qui apparaissent dans le cas d'un cadre seul que l'on retire tout le long de la périphérie du pansement dans un geste circulaire où l'action des forces de retrait est différente, ou d'une couche supplémentaire rigide après fixation sur la peau du patient.

En outre, la rigidification du pansement dans son état actuel résulte à la fois de la couche de liaison et des moyens de rigidification qui s'étendent au moins en partie sur la périphérie du pansement, mais laissent non recouverte la zone centrale de la couche de liaison. On obtient ainsi une rigidification différentielle du pansement. En effet, les moyens de rigidification, par exemple un cadre, procurent une rigidité plus importante que la couche de liaison et la couche de liaison assure dans la partie centrale du pansement une rigidification limitée mais suffisante pour permettre un lissage convenable du support sur la peau du patient en évitant la formation de plis. En revanche, les moyens de rigidification évitent que les bords du pansement ne se "replient".

Lorsque le pansement sert à fixer un cathéter ou un dispositif analogue sur la peau d'un patient, la rigidité dans la partie centrale du pansement est suffisamment réduite pour que le pansement, avec ses moyens de rigidification, se conforme à la forme du cathéter afin d'en assurer une fixation efficace, malgré la présence des moyens de rigidification qui sont limités à la périphérie du pansement.

Selon un mode préféré de mise en oeuvre de la présente invention le pansement possède une patte au niveau du cadre permettant de faciliter le retrait en invitant la personne qui pose le pansement à mettre en oeuvre un geste optimisé pour retirer la couche supplémentaire ce qui favorise encore l'absorption des forces exercées lors du retrait du système d'application.

De préférence également, les moyens de rigidification recouvrent au moins une partie de la périphérie de la couche de liaison en ménageant une fenêtre centrale. De préférence encore, ces moyens de rigidification forment un cadre.

Selon un autre mode préféré de mise en oeuvre de l'invention, le pansement comprend une couche absorbante qui couvre au moins en partie l'adhésif.

Selon un deuxième aspect de l'invention, le pansement comprend un support couvert au moins en partie sur la face de dessous d'un adhésif et sur la face de dessus d'un applicateur. Il se caractérise en ce que :
- la force de liaison entre le support (1) et l'applicateur est de 5 à 25 cN/cm, de préférence de 8 à 15 cN/cm ;
- la force de liaison du support (1) couvert au moins en partie de l'adhésif (2) sur plaque de verre est de 80 à 200 cN/cm, de préférence 100 à 150 cN/cm ; et
- la rigidité du pansement mesurée par la méthode de la flèche donne un angle α de 30 à 60°, préférentiellement de 35 à 55°.

### Description des figures

D'autres caractéristiques et avantages de l'invention apparaîtront mieux à la lecture de la description qui suit de plusieurs modes de réalisation de l'invention. La description se réfère aux figures annexées sur lesquelles.
- La figure 1 représente une vue de dessus d'un pansement selon la présente invention.
- La figure 2A représente une coupe transversale dans le sens I-I d'un premier mode de réalisation selon la présente invention dans lequel l'adhésif 6 couvre l'ensemble du cadre 5.
- La figure 2B représente une coupe transversale dans le sens I-I d'un second mode de réalisation selon la présente invention dans lequel l'adhésif 6 n'est présent que sur les parties A et B du cadre 5.
- La figure 3A représente une coupe transversale dans le sens II-II d'un troisième mode de réalisation de l'invention dans lequel la couche de protection 3 est plus longue que les autres couches formant le pansement.
- La figure 3B représente une coupe transversale dans le sens II-II d'un quatrième mode de réalisation selon la présente invention dans lequel on a un évidement de l'adhésif 6.
- La figure 4 représente une variante du pansement selon l'invention dans laquelle on note la présence d'une patte 11.
- La figure 5 représente une variante du pansement selon l'invention dans laquelle le pansement a une forme ovoïde.
- La figure 6 représente une variante du pansement selon l'invention dans laquelle le cadre ne couvre que 3 bords périphériques.
- La figure 7 représente une variante du pansement selon l'invention dans laquelle on note la présence d'une encoche permettant le passage de tubes ou cathéter.
- Les figures 8A, 8B, 8C et 8D sont des schémas explicatifs de l'application d'un pansement selon la présente invention.
- La figure 9 est un schéma explicatif de la méthode de mesure de rigidité (mesure de la flèche ou « bending test »).

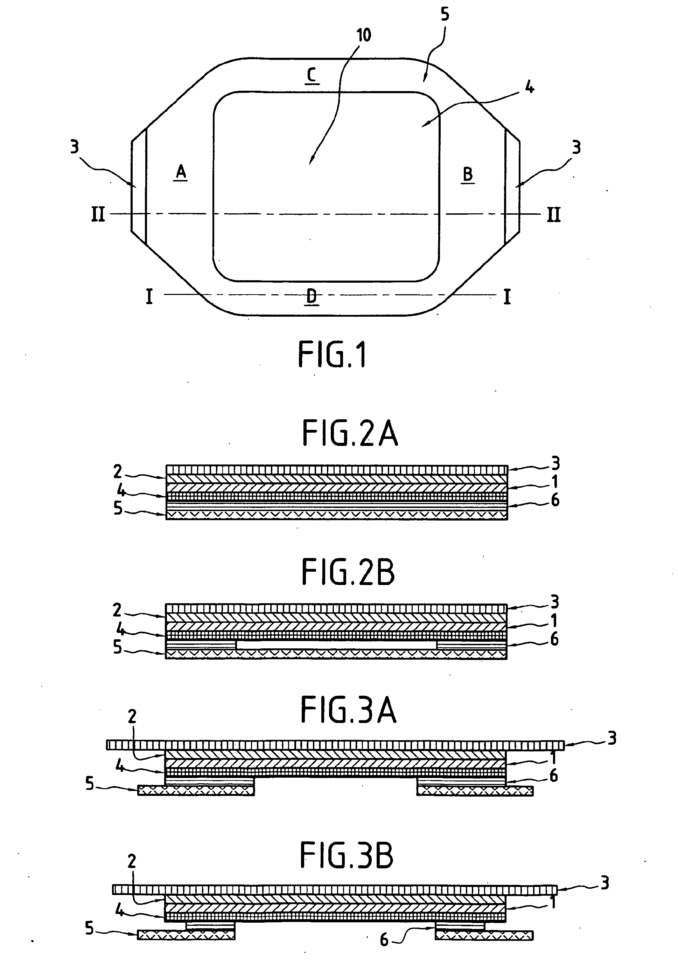

La structure d'un pansement composite adhésif selon la présente invention est illustrée en se référant aux figures 1, 2A et 2B qui représentent respectivement une vue du dessus et une coupe transversale dans le sens I-I du pansement.

Le pansement composite adhésif selon l'invention est constitué des différents éléments suivants :
- un support 1 formé de préférence d'un film mince, souple et flexible ayant une face du dessus et une face de dessous ;
- un adhésif 2 sensible à la pression appliqué sur au moins une partie de la face du dessous du support 1 ;
- une couche de protection 3 appliquée de façon détachable sur l'adhésif 2 sensible à la pression à l'opposé du support 1 ;
- une couche de liaison 4, comprenant une face de dessus et une face de dessous constituée de préférence d'un film mince et souple qui est fixée par sa face de dessous avec la face de dessus du support 1 à l'opposé de l'adhésif 2, ledit support 1 et la couche de liaison 4 étant rendus solidaires par tout moyen de fixation ;
- des moyens de rigidification qui, dans l'exemple décrit, sont constitués par un cadre 5 comprenant une face de dessus et une face de dessous qui est fixé au moins en partie sur la périphérie du pansement sur la face de dessus de la couche de liaison 4 par un moyen de fixation 6, ledit moyen 6 étant présent au moins en partie sur la périphérie de la face de dessus de la couche de liaison 4 correspondant au cadre 5. La couche de liaison 4 et les moyens de rigidification 5 forment l'applicateur.

Plus précisément, la figure 1 présente le pansement sous une vue de dessus dans laquelle on aperçoit le cadre 5 qui forme une fenêtre 10 dans laquelle apparaît la face de dessus de la couche de liaison 4. Les lignes pleines représentent les limites droite et gauche du cadre laissant apparaître le protecteur 3 qui, dans ce mode de réalisation est plus long que le cadre 5.

Selon une variante, on peut prévoir sur le pansement décrit ci-dessus que le moyen de fixation 6 est présent uniquement sur les deux côtés les plus larges A et B (représentés à la figure 1) entre le cadre 5 et la face de dessus de la couche de liaison 4.

Dans tous les cas, les moyens de rigidification ne recouvrent pas la zone centrale de la couche de liaison.

La figure 2A présente une coupe transversale dans le sens I-I où le support 1 est entièrement enduit de l'adhésif 2 qui est couvert de la couche de protection 3. La figure 2B représente une même coupe d'un pansement dans lequel le moyen de fixation 6 n'est présent que sur les parties larges A et B du cadre 5.

Comme on peut le constater sur la figure 3A qui représente une coupe transversale dans le sens II-II, le cadre 5 peut s'étendre au-delà de la surface définie par l'association des couches 1, 2 et 4 de façon à permettre après élimination de la couche de protection 3 le maniement du pansement sans toucher la couche d'adhésif 2.

Selon une autre version optimisée du pansement représentée sur la figure 3B on peut réaliser un pansement dans lequel on procède à un évidement de la couche d'adhésif 6 afin d'éviter un fluage de cet adhésif en cas de pression appliquée sur le pansement qui pourrait conduire à l'adhésion de ce dernier dans son emballage.

La face de dessous du cadre 5 sans adhésif qui s'étend ainsi au-delà des trois couches précédentes peut être fixée de façon détachable à la face de dessus de la couche de protection 3 qui déborde elle aussi des trois couches 1, 2 et 4 et permettre ainsi une séparation aisée de ces dernières avant application du pansement sur la peau.

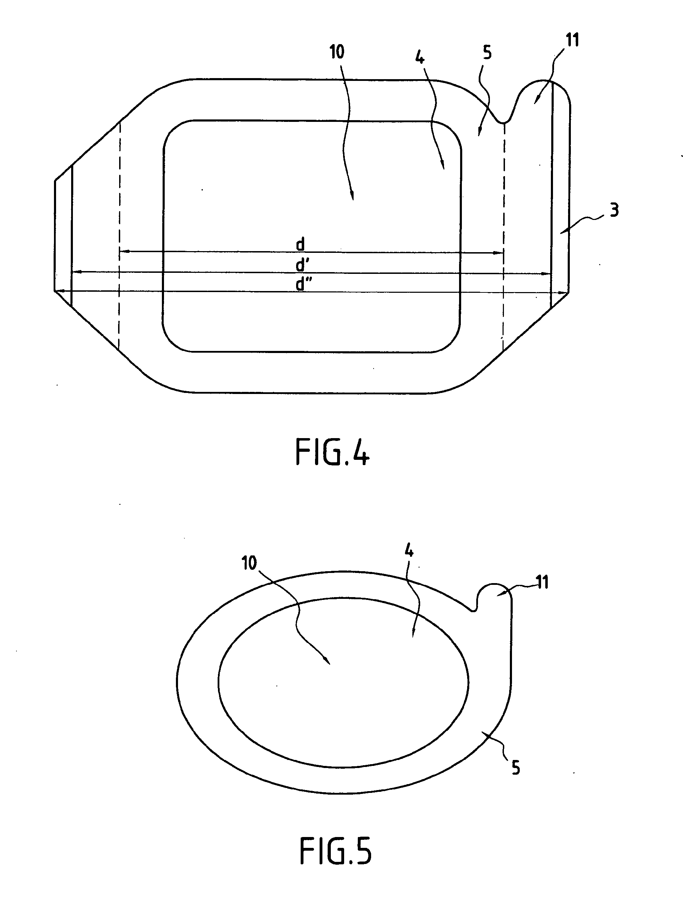

L'utilisation d'un pansement selon la présente invention est facilitée par la présence d'une patte 11 (figure 4): après retrait de la couche de protection couvrant l'adhésif, on fixe le pansement sur la peau par exemple puis on élimine l'ensemble constitué du cadre 5, de la couche supplémentaire 4 pour obtenir une fixation sans problème du pansement final constitué du support 1 et de l'adhésif 2.

Le pansement composite adhésif selon l'invention peut être de formes différentes : carré, rectangulaire (tel que représenté aux figures 1 et 4), ovoïde (tel que représenté à la figure 5) afin de mieux s'adapter à ses différentes applications.

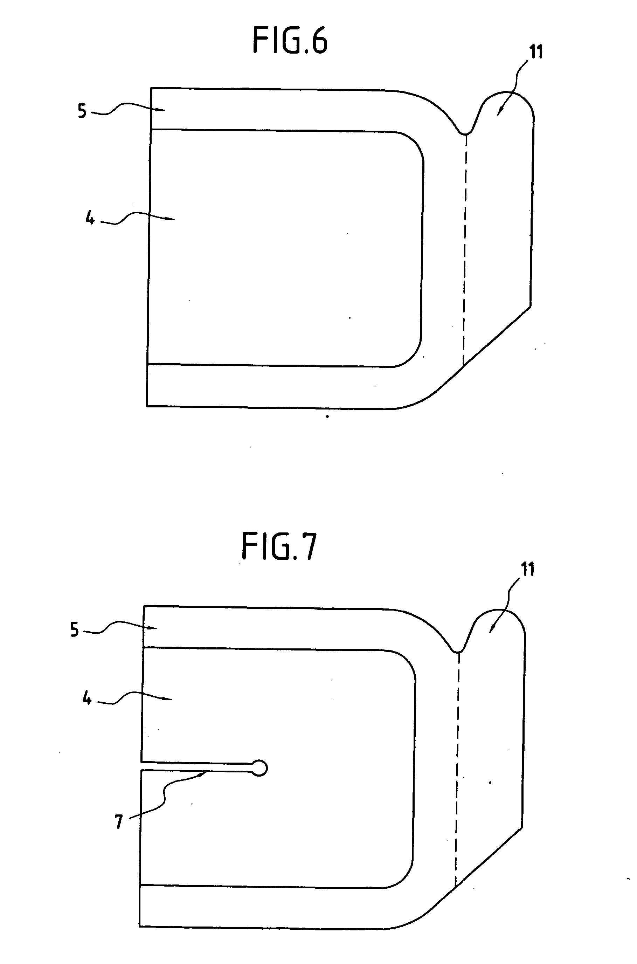

Dans une autre version du pansement composite adhésif selon l'invention représentée aux figures 6 et 7, le cadre 5 ne couvre pas l'ensemble de la périphérie du pansement, il peut, par exemple n'être constitué que de trois bords au lieu de quatre. Le pansement peut également présenter une encoche 7 permettant le passage de tubes et cathéters (figure 7).

La face du dessous du support 1 couvert au moins en partie de l'adhésif 2 peut éventuellement comprendre une couche absorbante. Cette couche absorbante est sélectionnée parmi le groupe se composant de textile à base de coton, rayonne, non-tissés, d'hydrocolloïdes, de mousses ou de combinaisons de ces éléments. Cette couche absorbante peut contenir une ou plusieurs substances sélectionnées parmi le groupe se composant d'agents antimicrobiens, de médicaments, d'indicateurs chimiques et de combinaisons de ces éléments. Si le pansement comprend une couche absorbante, celle-ci est préférentiellement positionnée sensiblement au milieu de la longueur totale du pansement.

La réalisation des pansements composites adhésifs selon l'invention met en oeuvre des constituants habituellement utilisés dans ce domaine.

Ainsi, la configuration du composite adhésif de la présente invention est utile en association avec n'importe quel support conformable comportant une enduction adhésive sensible à la pression appliquée sur le support. Des supports représentatifs englobent des textiles non-tissés, des textiles tissés, des textiles tricotés, des films et d'autres matières courantes servant de supports. Ce support est préférentiellement conformable aux surfaces anatomiques.

Le support 1 est, de préférence, un film mince, souple et flexible. Par mince, on entend un film ayant une épaisseur de 5 à 150 µm, de préférence de 15 à 70 µm. Par souple et flexible on entend tout matériau présentant une conformabilité suffisante pour s'adapter aux courbes du corps (telles que les articulations) ou au substrat (comme des cathéters par exemple). Ce film peut être respirant ou non. Il peut être étanche ou non. Parmi les films utilisables en tant que support 1 on préfère les films en polyuréthanes, en polyester, en polyamide, les films à base de copolymère de polyéther polyester (comme par exemple les produits commercialisés par la société DuPont sous la dénomination Hytrel®), à base de copolymères de polyester ou polyéther polyuréthanes (comme par exemple les produits commercialisés par la société Noveon sous la dénomination Estane®), à base de copolymères polyéther polyamide (comme par exemple les produits commercialisés par la société Arkema sous la dénomination Pebax@). D'autres polymères ou copolymères peuvent aussi être utilisés pour réaliser de tels films. On peut citer notamment les polyéthers, les polychlorures de vinyle, les polychlorures de vinylidène, les alcools polyvinyliques, les polyacétates de vinyles, les polystyrènes, les polyoléfines comme par exemple les polyéthylènes et les polypropylènes, les fluorures polyvinyliques, les copolymères triblocs ou diblocs de styrène et d'oléfine comme par exemple les styrène/butadiène (comme par exemple les produits commercialisés sous la dénomination Kraton®) et les polyéthers bloc amides. Des combinaisons de ces films peuvent être utilisées. De manière préférentielle, le support sera transparent ou translucide afin de faciliter la pose du pansement composite adhésif.

Les adhésifs 2 sensibles à la pression pouvant être utilisés dans la présente invention sont tous les adhésifs normalement utilisés pour leur application sur la peau, notamment les masses adhésives hypoallergéniques. Ces adhésifs sont décrits dans « Handbook of Pressure Sensitive Adhesive Technology », third Edition, Donatas Satas, chapter 13-22. On peut utiliser les adhésifs sensibles à la pression à base d'acrylique, de polyuréthane, de silicone, de caoutchouc naturel, de copolymère d'éthylène et d'acétate de vinyle ou de copolymères blocs du type poly(styrène-isoprène-styrène). Dans le cadre de la présente invention, on utilise de préférence les adhésifs acryliques sensibles à la pression en émulsion, phase solvant ou réticulables aux UV. De façon préférentielle, on utilisera les adhésifs acryliques sensibles à la pression réticulables UV comme par exemple les produits commercialisés par la société BASF sous la dénomination AcResin® A258UV).

Ces adhésifs sont appliqués sur le support 1 dans des quantités allant de 15 à 100 g/m².

L'adhésif sensible à la pression 2 peut éventuellement contenir une ou plusieurs substances sélectionnées parmi le groupe se composant d'agents antimicrobiens, de médicaments, ou toutes substances actives, d'indicateur d'infection, de substances allergènes, d'hydrocolloïdes pouvant absorber les exsudats et de combinaisons de ces éléments.

La couche de protection 3 peut être constituée de tout matériau de protection pelable couramment utilisée par l'homme du métier pour protéger la couche adhésive avant utilisation du pansement. Elle peut se présenter sous forme de film, par exemple un film de polyoléfine tel que le polyéthylène ou le polypropylène, un film de polyester, mais également d'une feuille métallique ou d'un papier siliconé.

La couche de liaison 4 peut être sélectionnée parmi le groupe se composant d'un film, d'une mousse, d'une grille. De manière préférentielle, la couche de liaison 4 sera transparente ou translucide afin de faciliter la pose du pansement composite adhésif. Cette couche de liaison 4 peut être en polyoléfine comme par exemple le polyéthylène ou le polypropylène. La couche de liaison 4 sera constituée, de préférence, d'un film de polyéthylène. L'épaisseur du film de polyéthylène est de 5 à 150 µm, de préférence 20 à 70 µm.

Pour faciliter la pose du pansement lorsque, par exemple, le support 1 est destiné à recouvrir un cathéter, cette couche de liaison 4 peut éventuellement présenter une rigidité diminuée au niveau de la fenêtre 10. Cette diminution de rigidité peut être obtenue par refente de la couche de liaison 4 ou par tout autre moyen connu de l'homme du métier permettant une variation de la rigidité à l'intérieur de la fenêtre.

La liaison entre la couche de liaison 4 et le support 1 peut être de nature physique, physico-chimique ou chimique. Le support 1 peut être thermocollé à la couche de liaison 4. De manière préférentielle, le support 1 et la couche de liaison 4 sont rendus solidaires par un processus d'extrusion bulle (ou extrusion gonflage). Dans ce procédé, la matière est extrudée grâce à une filière annulaire pour obtenir un tube qui est pincé entre des rouleurs étireurs. Une pression d'air est admise à l'intérieur de la gaine ainsi fermée, dans le but de l'étirer jusqu'à atteindre l'épaisseur requise. La "bulle" formée est refroidie par circulation d'air et enroulée. Dans la présente invention, on utilise de manière préférentielle un coextrudé bicouche composé d'un film de polyuréthane (qui constitue le support 1) et d'un film de polyéthylène (qui constitue la couche de liaison 4) obtenu par un processus d'extrusion bulle.

Le cadre 5 peut être constitué de tous matériaux capables d'apporter une certaine rigidité à la couche de liaison 4 ou à l'ensemble constitué du support 1, de l'adhésif 2 et de la couche de liaison 4 permettant ainsi une meilleure application en facilitant la pose du pansement composite selon l'invention. Parmi ces matériaux, on peut citer : le papier, le carton, une mousse, une grille, un non tissé...

Le cadre 5 peut également être créé par flocage sur la couche de liaison 4. Le cadre 5 peut ne couvrir qu'en partie la périphérie du pansement, son épaisseur et sa largeur peuvent également varier. Le cadre 5 est solidarisé à la couche de liaison 4 par un moyen de fixation 6. Le moyen de fixation 6 peut couvrir l'ensemble du cadre 5 ou n'être présent qu'en partie. On peut envisager un pansement dans lequel seules les deux parties les plus larges du cadre 5 sont solidarisées à la couche de liaison 4.

Le moyen de fixation 6 peut donc être tout moyen de fixation connu à ce jour conférant une force de liaison entre le cadre 5 et la couche de liaison 4 supérieure à celle existant entre la couche de liaison 4 et le support 1. De préférence, on utilisera un adhésif. Cet adhésif peut être choisi parmi tous les adhésifs connus de l'homme du métier.

Dans la présente invention, le cadre 5 et la couche de liaison 4 du pansement composite apportent de la rigidité à l'ensemble constitué de la couche support 1 et de l'adhésif 2, évitant ainsi au pansement de se recourber ou de se plier, même après retrait de la couche de protection 3. Cette particularité présente un avantage non négligeable, notamment au moment de la pose, lorsque l'opérateur retire la couche de protection, il peut maintenir le pansement à l'horizontale à l'aide d'une seule main avant de l'appliquer sur la peau ou sur le substrat auquel il est destiné (compresse, tube, cathéter...).

Dans la description qui précède, les moyens de rigidification sont constitués par un cadre 5 qui recouvre au moins trois côtés du support. Cependant, on ne sortirait pas de l'invention si les moyens de rigidification avaient une autre forme. Pour remplir leur fonction qui est de maintenir sensiblement plan le pansement, il faut que les moyens de rigidification aient une grande longueur et une grande largeur qui correspondent à la grande longueur et à la grande largeur du pansement pour maintenir correctement les bords de celui-ci.

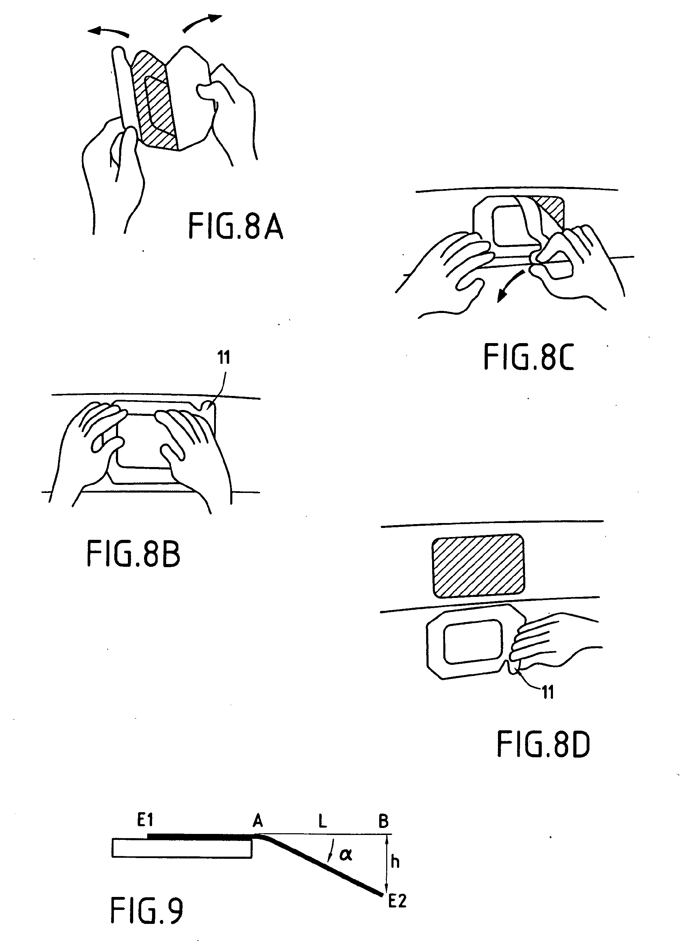

Le mode d'utilisation aisée du pansement est illustré sur les figures 8A, 8B, 8C, 8D. Le pansement est saisi par les deux bords libres du protecteur 3 et du cadre 5 puis le protecteur 3 est retiré (figure 8A). Après retrait de la couche de protection 3 le pansement peut être tenu à l'aide d'une seule main, puis il est appliqué sur le site auquel il est destiné sans toucher la couche adhésive 2 avec la main (figure 8B). Il suffit ensuite de retirer le cadre 5 auquel est solidarisé la couche de liaison 4 (figure 8C et 8D). Ce geste peut se faire à l'aide d'une éventuelle patte 11 présente au niveau du cadre 5, comme représentée à la figure 8D, facilitant ainsi le retrait du cadre 5 et de la couche de liaison 4. La liaison entre le cadre 5 et la couche de liaison 4 assurée par le moyen de fixation 6 est telle qu'aucune désolidarisation n'est possible lors du retrait de ces deux couches après la pose du pansement. De même, la force de décomplexage (ou force de liaison) existant entre le support 1 et la couche de liaison 4 est très inférieure au pouvoir adhésif existant entre le support 1, l'adhésif 2 et la peau ou le substrat auquel est destiné le pansement. De ce fait, les risques de décollement du pansement lors du retrait de la couche de liaison 4 et du cadre 5 sont minimisés.

D'autres caractéristiques et avantages de l'invention apparaîtront mieux à la lecture de la description de plusieurs modes de réalisation de l'invention.

Différents échantillons de pansements selon l'invention ont été réalisés en utilisant des techniques d'enduction classiques et de simples opérations de découpe selon le procédé suivant.

Le co-extrudé 1 constitué du film de polyuréthane solidarisé par un processus d'extrusion bulle à un film de polyéthylène est enduit selon une technique classique d'un adhésif 2 sensible à la pression. Cet adhésif 2 est couvert par une couche de protection 3. Puis, l'ensemble est découpé aux dimensions voulues. Le cadre 5 est enduit sur une de ces faces par un adhésif 6. La face adhésivée du cadre 5 est couverte d'un film protecteur. L'ensemble constitué du cadre 5, de l'adhésif 6 et du film protecteur est découpé aux dimensions voulues. Le film protecteur est ensuite retiré, puis l'ensemble constitué du cadre 5 et de l'adhésif 6 est positionné sur le film de polyéthylène 4.

Les produits ainsi réalisés sont décrits dans les exemples ci-dessous.

Lors de la réalisation de ces produits, on a fait varier :
■ Le grammage du matériau constituant le cadre 5
■ La largeur du cadre 5
■ L'épaisseur de la couche de liaison 4
■ La forme du pansement

Les différents matériaux utilisés sont les suivants :

Le cadre 5 est en papier ayant un grammage de 120 g/m² et une épaisseur de 100 µm ou 80 g/m² et une épaisseur de 110 µm. Le support 1 est en polyuréthane de 30 µm d'épaisseur et un grammage de 35 g/m², la couche de liaison 4 est en polyéthylène dont l'épaisseur est de 30 µm et un grammage de 40 g/m² ou 50 µm et un grammage de 46 g/m². L'adhésif 2 utilisé est un polyacrylate pur commercialisé par BASF sous la dénomination AcResin® A258UV à 40±3 g/m² pour tous ces exemples, l'adhésif 6 entre le cadre 5 et la couche de liaison 4 est une solution polyacrylate commercialisée par Solutia sous la dénomination Gelva® GMS 737 à 40 g/m².

Ces pansements sont de forme rectangulaire (tels que représentés à la figure 4). La dimension du support 1 et de la couche de liaison 4 est de 10*11.5 cm (longueur d), celle du cadre 5 est de 10*14.5 cm (longueur d'), celle du protecteur 3 est de 10*15.5 cm (longueur d").

Différents tests ont été réalisés sur différents pansements selon l'invention et les pansements commercialisés sous les dénominations TEGADERM® (société 3M, St. Paul, MN) et OPSITE® (société T.J. Smith & Nephew, Hull, Angleterre). Les produits TEGADERM® et OPSITE® sont décrits dans les exemples 15 et 16.

Les protocoles de réalisation des tests sont les suivants :

### Méthode de mesure de rigidité

La méthode de mesure de la flèche ou « bending test », telle que représentée à la figure 9 a été utilisée afin de mesurer la rigidité des pansements selon l'invention. Pour cette mesure, les pansements sont débarrassés de leurs couches de protection de l'adhésif et posés sur le rebord d'une paillasse 12 de sorte que la longueur de la partie hors de la paillasse soit de 11 cm. L'extrémité E1 du pansement est l'extrémité qui adhère à la paillasse, l'extrémité E2 du pansement est celle qui se trouve dans le vide. Le point A correspond au bord de la paillasse sur laquelle le pansement adhère, le point B correspond à E2 ramené à la verticale sur la droite (E1A). On mesure la longueur L représentant la distance entre les points A et B. La hauteur h représente la distance entre l'extrémité E2 du pansement et le point B. L'angle α est calculé comme suit : tanα=h/L. Dans cette cette méthode, plus l'angle α est faible, plus le matériau est considéré comme rigide. Les mesures sont effectuées à 21±2°C et à une humidité relative de 60±15%. Les exemples 1 à 13, 15 et 16 ont ainsi été testés.

### Méthode de mesure des forces de liaisons

Des mesures de décomplexage et d'adhésivité sur plaque de verre du pansement selon l'invention ont été réalisées. Pour ce faire, les pansements ont été découpés en éprouvette de 20 mm de largeur. Ces éprouvettes sont posées sur une plaque de verre. On effectue alors deux aller-retour avec un rouleau de masse M = 2 Kg/cm de largeur du produit. On laisse climatiser les éprouvettes à 21±2°C et à une humidité relative 61% pendant 10 minutes.

La force de décomplexage (ou force de liaison) de la couche de liaison 4 au support 1 ainsi que le pouvoir adhésif du support 1 et l'adhésif 2 (ou force de liaison) à la plaque de verre sont mesurés successivement au moyen d'un système électronique capable d'enregistrer une force par rapport à un déplacement (Synergie@ 200, Adamel). Les mesures sont effectuées avec un capteur de 10 N à une vitesse de 100 mm/min. Les exemples 14, 15 et 16 ont ainsi été testés.

### Ex. 1 :

Le grammage du papier servant à l'élaboration du cadre 5 est de 120 g/m², la largeur de celui-ci est de 20 mm. L'épaisseur de la couche de liaison 4 en polyéthylène est de 50 µm. Le pansement est de forme rectangulaire (telle que représentée à la figure 1).

### Ex. 2:

Le grammage du papier servant à l'élaboration du cadre 5 est de 120 g/m², la largeur de celui-ci est de 20 mm. L'épaisseur de la couche de liaison 4 en polyéthylène est de 30 µm. Le pansement est de forme rectangulaire (telle que représentée à la figure 1).

### Ex. 3 :

Le grammage du papier servant à l'élaboration du cadre 5 est de 80 g/m², la largeur de celui-ci est de 20 mm. L'épaisseur de la couche de liaison 4 en polyéthylène est de 50 µm. Le pansement est de forme rectangulaire (telle que représentée à la figure 1).

### Ex. 4:

Le grammage du papier servant à l'élaboration du cadre 5 est de 80 g/m², la largeur de celui-ci est de 20 mm. L'épaisseur de la couche de liaison 4 en polyéthylène est de 30 µm. Le pansement est de forme rectangulaire (telle que représentée à la figure 1).

### Ex. 5 :

Le grammage du papier servant à l'élaboration du cadre 5 est de 120 g/m², la largeur de celui-ci est de 12 mm. L'épaisseur de la couche de liaison 4 en polyéthylène est de 50 µm. Le pansement est de forme rectangulaire (telle que représentée à la figure 1).

### Ex. 6 :

Le grammage du papier servant à l'élaboration du cadre 5 est de 80 g/m², la largeur de celui-ci est de 12 mm. L'épaisseur de la couche de liaison 4 en polyéthylène est de 30 µm. Le pansement est de forme rectangulaire (telle que représentée à la figure 1).

### Ex. 7 :

Le grammage du papier servant à l'élaboration du cadre 5 est de 80 g/m², la largeur de celui-ci est de 12 mm. L'épaisseur de la couche de liaison 4 en polyéthylène est de 50 µm. Le pansement est de forme rectangulaire (telle que représentée à la figure 1).

### Ex. 8 :

Le grammage du papier servant à l'élaboration du cadre 5 est de 120 g/m², la largeur de celui-ci est de 12 mm. L'épaisseur de la couche de liaison 4 en polyéthylène est de 30 µm. Le pansement est de forme rectangulaire (telle que représentée à la figure 1).

### Ex. 9 :

Le grammage du papier servant à l'élaboration du cadre 5 est de 120 g/m², la largeur de celui-ci est de 8 mm. L'épaisseur de la couche de liaison 4 en polyéthylène est de 30 µm. Le pansement est de forme rectangulaire (telle que représentée à la figure 1).

### Ex. 10 :

Le grammage du papier servant à l'élaboration du cadre 5 est de 120 g/m², la largeur de celui-ci est de 8 mm. L'épaisseur de la couche de liaison 4 en polyéthylène est de 50 µm. Le pansement est de forme rectangulaire (telle que représentée à la figure 1).

### Ex. 11 :

Le grammage du papier servant à l'élaboration du cadre 5 est de 80 g/m², la largeur de celui-ci est de 8 mm. L'épaisseur de la couche de liaison 4 en polyéthylène est de 50 µm. Le pansement est de forme rectangulaire (telle que représentée à la figure 1).

### Ex. 12 :

Le grammage du papier servant à l'élaboration du cadre 5 est de 80 g/m², la largeur de celui-ci est de 8 mm. L'épaisseur de la couche de liaison 4 en polyéthylène est de 30 µm. Le pansement est de forme rectangulaire (telle que représentée à la figure 1).

### Ex. 13 :

Le grammage du papier servant à l'élaboration du cadre 5 est de 80 g/m², la largeur de celui-ci est de 12 mm. L'épaisseur de la couche de liaison 4 en polyéthylène est de 30 µm. Le pansement est de forme ovale (telle que représentée à la figure 5).

### Ex. 14 :

Le support 1 de l'éprouvette est en polyuréthane d'une épaisseur de 30 µm et la couche de liaison 4 en polyéthylène d'une épaisseur de 30 µm, ces deux couches étant solidarisées par un processus d'extrusion bulle. L'adhésif 2 est la masse AcResin A258UV de BASF, le grammage de cette masse étant de 40±3g/m² et la réticulation de 50mJ/cm. Le cadre 5 est en papier de 80g/m², l'adhésif 6 est Gelva GMS 737 de Solutia à 40 g/m².

### Ex. 15 :

Le pansement OPSITE® 10*12 cm de Smith & Nephew est constitué d'un film de polyuréthane d'une épaisseur de 27 µm et d'un grammage de 28 g/m² et d'un liner de polyéthylène d'une épaisseur de 70 µm et d'un grammage de 56 g/m². L'adhésif a un grammage de 29 g/m².

### Ex. 16 :

Le pansement TEGADERM® 10*12 cm de 3M est constitué d'un film de polyuréthane d'une épaisseur de 22 µm et d'un grammage de 25 g/m² et d'un cadre en papier ayant une épaisseur de 120 µm et un grammage de 53 g/m². L'adhésif a un grammage de 20 g/m².

Les résultats des mesures de rigidité sont rassemblés dans le tableau I.

**Tableau I : mesures de rigidité**

| **Essais** | | **Angle α** |
|---|---|---|
| **Ex. 1** | 120 g/m² | 35° |
| | 20 mm | |
| | PE 50 µm | |
| **Ex. 2** | 120 g/m² | 36° |
| | 20 mm | |
| | PE 30 µm | |
| **Ex. 3** | 80 g/m² | 38° |
| | 20 mm | |
| | PE 50 µm | |
| **Ex. 4** | 80 g/m² | 39° |
| | 20 mm | |
| | PE 30 µm | |
| **Ex. 5** | 120 g/m² | 43° |
| | 12 mm | |
| | PE 50 µm | |
| **Ex. 6** | 80 g/m² | 48° |
| | 12 mm | |
| | PE 50 µm | |
| **Ex. 7** | 80 g/m² | 48° |
| | 12 mm | |
| | PE 50 µm | |
| **Ex. 8** | 120 g/m² | 48° |
| | 12 mm | |
| | PE 30 µm | |
| **Ex. 9** | 120 g/m² | 51° |
| | 8 mm | |
| | PE 30 µm | |
| **Ex. 10** | 120 g/m² | 50° |
| | 8 mm | |
| | PE 50 µm | |
| **Ex. 11** | 80 g/m² | 52° |
| | 8 mm | |
| | PE 50 µm | |
| **Ex. 12** | 80 g/m² | 54° |
| | 8 mm | |
| | PE 30 µm | |
| **Ex. 13** | 80 g/m² | 37° |
| | 12 mm | |
| | PE 30 µm | |
| **Ex. 15** | OPSITE | > 70° |
| **Ex. 16** | TEGADERM | > 70° |

Les résultats obtenus mettent en évidence une rigidité très inférieure des produits OPHITE® et TEGADERM® comparée aux pansements selon l'invention. Les différences entre les valeurs d'angle sont de l'ordre de 10 à 35° entre les pansements selon l'invention et ces deux produits. L'angle α n'a pu être mesuré avec précision du fait du manque de rigidité pour ces deux pansements. De plus, le pansement TEGADERM® a tendance à se recourber et à garder cette déformation. On constate donc que seuls les pansements selon l'invention présentent une meilleure rigidité après retrait du protecteur.

Les pansements composites adhésifs selon la présente invention ont un angle α (obtenu selon cette méthode de la flèche) compris entre 30 et 60°, de préférence de 35 à 55°.

Les résultats des mesures de forces de liaison sont rassemblés dans le tableau II.

**Tableau II : mesures de forces de liaison**

| | Ex. 14 | OPSITE | TEGADERM |
|---|---|---|---|
| Pouvoir adhésif (cN/cm) | 159±4 | 156±10 | 82±2 |
| Force de décomplexage (cN/cm) | 9.6±1.8 | 24±1 | 28±7 |
| Rapport Force de décomplexage/Pouvoir adhésif | 0.06 | 0.15 | 0.34 |

Le pansement selon l'invention présente une force de décomplexage faible comparée à son pouvoir adhésif évitant ainsi toute perturbation de la liaison adhésive entre le pansement et la peau ou tout matériau sur lequel le pansement est appliqué lors du retrait du cadre 5 et de la couche de liaison 4. Ainsi le rapport entre la force de décomplexage et le pouvoir adhésif du pansement selon l'invention est très inférieur à celui obtenu avec les pansements OPSITE® et TEGADERM®. Les pansements composites adhésifs selon l'invention présentent une force de liaison du support 1 couvert au moins en partie de l'adhésif 2 sur plaque de verre de 80 à 200 cN/cm, de préférence 100 à 150 cN/cm et une force de liaison entre le support 1 et la couche de liaison 4 est de 5 à 25 cN/cm, de préférence 8 à 15 cN/cm.

Ainsi les pansements composites adhésifs selon l'invention présentent une force de décomplexage faible tout en ayant une meilleure rigidité. Les problèmes d'enroulement du pansement sur lui-même ainsi que le décollement du support lors du retrait de la couche de matériau rigide après application du pansement sur la peau sont écartés.

### Mode de réalisation préférentiel d'un pansement selon l'invention

Le pansement selon l'invention est préférentiellement constitué d'un support 1 qui est un film en polyuréthane d'une épaisseur de 30 µm dont la face de dessous est couverte d'une masse adhésive AcResin A258UV de BASF dont le grammage est de 40±3 g/m² et la réticulation de 50mJ/cm. La couche de protection 3 est en papier siliconé. La couche de liaison 4 est en polyéthylène d'une épaisseur de 30 µm, le coextrudé support 1 - couche de liaison 4 est obtenu par un processus d'extrusion bulle. Le cadre 5 est en papier (120 g/m²), la largeur au niveau des segments C et D (représentés sur la figure 1) est de 120 mm. Il est fixé au support au moyen de l'adhésif 6 (Gelva GMS 737 de Solutia à 40 g/m²). Comme indiqué sur la figure 4, le pansement possède une patte 11 facilitant le retrait de la couche de liaison 4 et du cadre 5 après la pose du pansement.

Les pansements selon l'invention peuvent se présenter sous forme de pansements individuels de petite dimension ou de dimension plus importante selon l'utilisation qui en est faite. Ces pansements seront alors conditionnés individuellement dans une enveloppe scellée assurant une conservation en milieu stérile.

## Revendications

1. Pansement composite adhésif comprenant :
- un support (1) constitué d'un film mince et souple ayant une face de dessus et une face de dessous ;
- un adhésif (2) sensible à la pression appliqué sur au moins une partie de la face du dessous du support (1) ;
- une couche de protection (3) appliquée de façon détachable sur l'adhésif (2) sensible à la pression à l'opposé du support (1) ;
**caractérisé en ce qu'**il comporte en outre :
- une couche de liaison (4) comprenant une face de dessus et une face de dessous qui est assemblée de façon détachable par sa face de dessous à la face de dessus du support (1) à l'opposé de l'adhésif (2) ; et
- des moyens de rigidification (5) comprenant une face de dessus et une face de dessous, et étant fixés au moins en partie sur la périphérie du pansement sur la face de dessus de la couche de liaison (4) par un moyen de fixation (6), en laissant la zone centrale de ladite couche de liaison (4) non recouverte par lesdits moyens de rigidification (5), lesdits moyens de rigidification (5) et ladite couche de liaison (4) apportant une rigidité au support (1),
la force de liaison entre le support (1) couvert au moins en partie par l'adhésif (2) et la peau étant supérieure à la force de liaison entre le support (1) et la couche de liaison (4), et la force de liaison entre les moyens de rigidification (5) et la couche de liaison (4) étant elle aussi supérieure à la force de liaison entre la couche de liaison (4) et le support (1).

2. Pansement composite adhésif selon la revendication 1, dans lequel lesdits moyens de rigidification (5) recouvrent au moins une partie de la périphérie dudit pansement en ménageant une fenêtre (10) dans ladite zone centrale.

3. Pansement composite adhésif selon la revendication 2, dans lequel lesdits moyens de rigidification (5) recouvrent l'intégralité de la périphérie du pansement en constituant un cadre.

4. Pansement composite adhésif selon l'une quelconque des revendications 1 à 3, dans lequel la force de liaison du support (1) couvert au moins en partie de l'adhésif (2) sur plaque de verre est de 80 à 200 cN/cm, de préférence 100 à 150 cN/cm et la force de liaison entre le support (1) et la couche de liaison (4) est de 5 à 25 cN/cm, de préférence 8 à 15 cN/cm.

5. Pansement composite adhésif selon l'une quelconque des revendications précédentes, dans lequel la rigidité mesurée par la méthode de mesure de la flèche donne un angle α de 30 à 60°, préférentiellement de 35 à 55°.

6. Pansement composite adhésif selon l'une quelconque des revendications précédentes, dans lequel le support (1) est sélectionné parmi le groupe se composant d'un film en polyuréthane, polyester, polyamide.

7. Pansement composite adhésif selon l'une quelconque des revendications précédentes, dans lequel la couche de liaison (4) est constituée de matériau de type polyoléfine, de préférence un film de polyéthylène.

8. Pansement composite adhésif selon l'une quelconque des revendications précédentes, dans lequel les moyens de rigidification sont fixés à la couche de liaison (4) par un adhésif ou tout autre moyen de fixation (6) ou formé directement sur la couche de liaison (4), par exemple, par flocage.

9. Pansement composite adhésif selon l'une quelconque des revendications précédentes, dans lequel les moyens de rigidification sont en papier, carton, mousse, grille ou tout autre matériau capable d'apporter une rigidité à la couche de liaison (4) ou à l'ensemble constitué du support (1), de l'adhésif (2) et de la couche de liaison (4) une rigidité supérieure à celle de la couche de liaison (4).

10. Pansement composite adhésif selon l'une quelconque des revendications précédentes, dans lequel la face du dessous du support (1) couvert au moins en partie de l'adhésif (2) comprend une couche absorbante.

11. Pansement composite adhésif selon l'une quelconque des revendications précédentes, dans lequel on utilise dans le pansement un coextrudé bicouche composé de polyuréthane et polyéthylène rendus solidaires par un processus d'extrusion bulle.

## Patentansprüche

1. Mehrlagiges Heftpflaster, umfassend:
- einen Träger (1), der aus einer dünnen und flexiblen Folie mit einer Ober- und einer Unterseite besteht,
- ein Heftband (2), das für den Druck empfindlich ist, der auf mindestens einen Teil der Unterseite des Trägers (1) ausgeübt wird,
- eine Schutzschicht (3), die lösbar auf das für den Druck empfindliche Heftband (2) gegenüber dem Träger (1) aufgebracht ist;
**dadurch gekennzeichnet, dass** es ferner umfasst:
- eine Verbindungsschicht (4), umfassend eine Oberseite und eine Unterseite, die lösbar mit ihrer Unterseite an der Oberseite des Trägers (1) gegenüber dem Heftband (2) angeordnet ist, und
- Versteifungsmittel (5), umfassend eine Oberseite und eine Unterseite, die zumindest teilweise an der Peripherie des Pflasters auf der Oberseite der Verbindungsschicht (4) durch ein Befestigungsmittel (6) befestigt sind, wobei die zentrale Zone der Verbindungsschicht (4) von den Versteifungsmitteln (5) unbedeckt bleibt, wobei die Versteifungsmittel (5) und die Verbindungsschicht (4) dem Träger (1) eine Steifigkeit verleihen,
wobei die Verbindungskraft zwischen dem Träger (1), der zumindest teilweise mit dem Heftband (2) bedeckt ist, und der Haut größer als die Verbindungskraft zwischen dem Träger (1) und der Verbindungsschicht (4) ist, und wobei die Verbindungskraft zwischen den Versteifungsmitteln (5) und der Verbindungsschicht (4) ebenfalls größer als die Verbindungskraft zwischen der Verbindungsschicht (4) und dem Träger (1) ist.

2. Mehrlagiges Heftpflaster nach Anspruch 1, bei dem die Versteifungsmittel (5) zumindest einen Teil der Peripherie des Pflasters bedecken, wobei ein Fenster (10) in der zentralen Zone freigelassen wird.

3. Mehrlagiges Heftpflaster nach Anspruch 2, bei dem die Versteifungsmittel (5) die Gesamtheit der Peripherie des Pflasters bedecken, wobei sie einen Rahmen bilden.

4. Mehrlagiges Heftpflaster nach einem der Ansprüche 1 bis 3, bei dem die Verbindungskraft des Trägers (1), der zumindest teilweise mit dem Heftband (2) bedeckt ist, auf Glasplatte 80 bis 200 cN/cm, vorzugsweise 100 bis 150 cN/cm beträgt, und die Verbindungskraft zwischen dem Träger (1) und der Verbindungsschicht (4) 5 bis 25 cN/cm, vorzugsweise 8 bis 15 cN/cm beträgt.

5. Mehrlagiges Heftpflaster nach einem der vorhergehenden Ansprüche, bei dem Steifigkeit, gemessen durch die Pfeilmessmethode, einen Winkel ά von 30 bis 60°, vorzugsweise von 35 bis 55°, ergibt.

6. Mehrlagiges Heftpflaster nach einem der vorhergehenden Ansprüche, bei dem der Träger (1) in der Gruppe, bestehend aus Polyurethan, Polyester, Polyamid, ausgewählt wird.

7. Mehrlagiges Heftpflaster nach einem der vorhergehenden Ansprüche, bei dem die Verbindungsschicht (4) aus einem Material des Typs Polyolefin, vorzugsweise einer Polyäthylenfolie, gebildet ist.

8. Mehrlagiges Heftpflaster nach einem der vorhergehenden Ansprüche, bei dem die Versteifungsmittel an der Verbindungsschicht (4) durch ein Heftband oder jedes andere Befestigungsmittel (6) befestigt oder direkt auf der Verbindungsschicht (4), beispielsweise durch Beflocken, ausgebildet ist.

9. Mehrlagiges Heftpflaster nach einem der vorhergehenden Ansprüche, bei dem die Versteifungsmittel aus Papier, Karton, Schaum, Gitter oder jedem anderen Material bestehen, das geeignet ist, der Verbindungsschicht (4) eine Steifigkeit zu verleihen, oder der Einheit, bestehend aus dem Träger (1), dem Heftband (2) und der Verbindungsschicht (4) eine größere Steifigkeit als jene der Verbindungsschicht (4) zu verleihen.

10. Mehrlagiges Heftpflaster nach einem der vorhergehenden Ansprüche, bei dem die Unterseite des Trägers (1), die zumindest teilweise mit dem Heftband (2) bedeckt ist, eine absorbierende Schicht umfasst.

11. Mehrlagiges Heftpflaster nach einem der vorhergehenden Ansprüche, bei dem für das Pflaster ein zweischichtiges Co-Extrudat aus Polyurethan und Polyäthylen, die durch ein Bubble-Extrusionsverfahren verbunden werden, verwendet wird.

## Claims

1. A composite adhesive dressing comprising:
· a support (1) constituted by a thin pliable film with a top face and a bottom face;
· a pressure-sensitive adhesive (2) applied to at least a portion of the bottom face of the support (1);
· a protective layer (3) applied detachably to the pressure-sensitive adhesive (2) opposite the support (1); **characterized in that** it further comprises:
· a bonding layer (4) comprising a top face and a bottom face that is detachably assembled via its bottom face to the top face of the support (1) opposite the adhesive (2); and
· rigidification means (5) comprising a top face and a bottom face, and being fixed at least in part to the periphery of the dressing on the top face of the bonding layer (4) via a fastener means (6), leaving the central zone of said bonding layer (4) not covered by said rigidification means (5), said rigidification means (5) and said bonding layer (4) providing the support (1) with rigidity,
the bonding force between the support (1) that is covered at least in part with the adhesive (2) and the skin being greater than the bonding force between the support (1) and the bonding layer (4), and the bonding force between the rigidification means (5) and the bonding layer (4) also being greater than the bonding force between the bonding layer (4) and the support (1).

2. A composite adhesive dressing according to claim 1, in which said rigidification means (5) cover at least a portion of the periphery of said dressing, forming an aperture (10) in said central zone.

3. A composite adhesive dressing according to claim 2, in which said rigidification means (5) cover the whole of the periphery of the dressing, constituting a frame.

4. A composite adhesive dressing according to any one of claims 1 to 3, in which the glass plate bonding force of the support (1) that is covered at least in part with adhesive (2) is 80 cN/cm to 200 cN/cm, preferably 100 cN/cm to 150 cN/cm, and the bonding force between the support (1) and the bonding layer (4) is 5 cN/cm to 25 cN/cm, preferably 8 cN/cm to 15 cN/cm.

5. A composite adhesive dressing according to any preceding claim, in which the rigidity angle ·, measured by the bending test method, is 30° to 60°, preferably 35° to 55°.

6. A composite adhesive dressing according to any preceding claim, in which the support (1) is selected from the group composed of a film of polyurethane, polyester or polyamide.

7. A composite adhesive dressing according to any preceding claim, in which the bonding layer (4) is constituted by a polyolefin type material, preferably a film of polyethylene.

8. A composite adhesive dressing according to any preceding claim, in which the rigidification means are fixed to the bonding layer (4) by an adhesive or any other fastener means (6) or formed directly on the bonding layer (4), for example by flock coating.

9. A composite adhesive dressing according to any preceding claim, in which the rigidification means are formed from paper, card, foam, mesh or any other material that is capable of endowing the bonding layer (4) or the assembly constituted by the support (1), the adhesive (2) and the bonding layer (4) with rigidity that is greater than that of the bonding layer (4).

10. A composite adhesive dressing according to any preceding claim, in which the bottom face of the support (1) that is covered at least in part with the adhesive (2) comprises an absorbant layer.

11. A composite adhesive dressing according to any preceding claim, in which a co-extruded bilayer is used in the dressing, composed of polyurethane and polyethylene attached together using a blown-bubble extrusion procedure.
